# FASCICULE DE BREVET EUROPEEN

(11) **EP 0 885 626 B1**
(45) Date de publication et mention de la délivrance du brevet: **13.10.2004**
(21) Numéro de dépôt: 98401532.1
(22) Date de dépôt: 22.06.1998
(51) Int. Cl.: A61N 1/362

(54) **Dispositif et procédé pour le traitement des troubles du rythme auriculaire**
Gerät und Verfahren zur Behandlung atrialer Arrhythmien
System and method for treating atrial arrhythmia

(30) Priorité: 20.06.1997 FR 9707672
(43) Date de publication de la demande: 23.12.1998
(73) Titulaire: ELA MEDICAL (Société anonyme), 92541 Montrouge (FR)
(72) Inventeur: Bonhour, Anne, 92410 Ville d'Avray (FR); Limousin, Marcel, 75014 Paris (FR); Bonnet, Jean-Luc, 92120 Montrouge (FR)
(74) Mandataire: Dupuis-Latour, Dominique

(56) Documents cités:
- EP-A- 0 488 841
- EP-A- 0 488 904
- WO-A-98/25670
- US-A- 5 226 415
- US-A- 5 403 356

## Description

L'invention concerne un dispositif médical implantable actif, en particulier un dispositif de la famille des stimulateurs, des défibrillateurs et des cardioverteurs pourvus d'une fonction de stimulation double chambre pour le traitement des troubles du rythme auriculaire.

De tels dispositifs sont définis, par exemple, dans la directive 90/385/CEE du 20 juin 1990 du Conseil des communautés européennes. On trouve parmi ces dispositifs des appareils dits "double chambre" dans la mesure où ils recueillent et délivrent des signaux dans les cavités haute (l'oreillette) et basse (le ventricule) du coeur. Ces dispositifs sont conçus pour suivre le rythme cardiaque du patient et effectuer certaines fonctions de diagnostic et/ou de thérapie des arythmies auriculaires (AA) et/ou ventriculaires (AV).

Un tel stimulateur cardiaque en cas d'extrasystole ventriculaire (ESV) est décrit, par exemple, dans la demande de brevet EP-A-0 550 342. On décrit dans ce document un moyen de prévention des troubles du rythme cardiaque qui peuvent survenir à l'apparition d'une extrasystole ventriculaire (ESV). Une extrasystole ventriculaire peut induire une pause pouvant favoriser l'établissement d'un trouble du rythme par la désynchronisation des périodes réfractaires ventriculaires. Dans ces conditions, une tachycardie peut s'établir.

Il n'est pas possible de prévoir la survenue d'une extrasystole ventriculaire, mais il est possible grâce au procédé décrit dans la demande de brevet EP-A-0 550 342 de prévenir les conséquences néfastes d'un tel événement.

Selon ce dispositif de l'état de l'art, sur la détection d'une extrasystole, on stimule l'oreillette de façon concomitante à l'apparition de l'extrasystole ventriculaire, on stimule ensuite l'oreillette à un rythme plus rapide que celui existant avant l'apparition de l'extrasystole ventriculaire, on diminue le délai auriculo-ventriculaire (AV) lors de cette stimulation rapide et on revient par paliers lentement vers la fréquence de base préexistante avant l'apparition de l'extrasystole ventriculaire. La pause ventriculaire est ainsi évitée.

Cette technique a été mise en oeuvre à l'aide de programmes téléchargés, en particulier dans des appareils de la gamme *Chorus* fabriqués par la société Ela Médical.

Toutefois, certaines limitations sont apparues à l'observation des enseignements de la clinique. Un des inconvénients du dispositif susmentionné est qu'il ne traite que les extrasystoles d'origine ventriculaire et ne s'occupe pas des extrasystoles auriculaires (ESA) donnant lieu au même phénomène de désynchronisation, préjudiciables à une bonne stabilité du rythme cardiaque. Par ailleurs, lors de la survenue de doublets ou triplets d'extrasystoles qui sont, respectivement, une suite de deux extrasystoles (ESA ou ESV) ou une suite de trois extrasystoles sans événement non extrasystolique intermédiaire, le dispositif est désactivé et ne traite pas spécifiquement ce genre d'événement cardiaque.

De plus, il a été noté que la variation du rythme lors de la phase d'accélération après la détection d'une extrasystole ventriculaire n'était pas suffisamment physiologique car elle ne tient pas compte de la précocité de l'extrasystole. Et, lors de l'apparition répétée d'une extrasystole ventriculaire durant l'activation de l'algorithme du procédé, celui-ci a tendance à accélérer le rythme à une fréquence de plus en plus élevée, typiquement jusqu'à la fréquence maximale programmée. Une telle accélération successive qui est normalement favorable pose le problème que très souvent la fréquence de stimulation ne correspond pas aux besoins physiologiques du patient. Par exemple, une accélération faisant passer la fréquence de 100 à 120 cpm n'est pas problématique mais une accélération à partir de 50 cpm jusqu'à une fréquence maximale (typiquement de 120 cpm) n'est pas appropriée si le patient est au repos.

Le but de la présente invention est de proposer un dispositif pour prévenir des troubles du rythme auriculaire susceptibles d'apparaître après la survenue d'une ou plusieurs extrasystoles auriculaires, procédé dont la caractéristique principale est de prendre en compte non seulement une extrasystole auriculaire isolée mais aussi des extrasystoles auriculaires apparaissant sous la forme de doublets ou de triplets.

Un autre but de la présente invention est une meilleure adaptation de l'accélération du rythme cardiaque après la détection d'une extrasystole auriculaire (isolée ou multiple) en tenant compte du couplage de cette extrasystole auriculaire avec l'événement auriculaire précédant son apparition.

De plus, encore un autre but de la présente invention est que la fréquence maximale vers laquelle tend le dispositif en cas de réactivation de l'algorithme de façon successive tienne compte de la fréquence moyenne du rythme sinusal antérieur. Dans le cas d'un appareil asservi, le dispositif peut aussi tenir compte de la fréquence moyenne indiquée par le capteur dont est muni le dispositif pour évaluer la fréquence maximale d'accélération.

A cet effet le dispositif médical implantable actif selon l'invention comprend des moyens de détection des signaux d'activité cardiaque auriculaire, des moyens de stimulation cardiaque dans l'oreillette et le ventricule du coeur, des moyens de détection de la survenue d'extrasystoles auriculaires et des moyens pour déclencher un intervalle d'échappement auriculaire intermédiaire appliqué lors de la détection d'une extrasvstole auriculaire.

Les documents WO-A-98/25670, EP-A-0 488 841 et US-A-5 226 415 divulguent chacun un dispositif selon le préambule de la revendication 1. WO-A-98/25670 appartient à l'état de la technique selon l'article 54(3) CBE.

De façon caractéristique de l'invention, l'intervalle d'échappement auriculaire intermédiaire est une fonction du couplage de l'extrasystole auriculaire détectée par rapport à l'événement auriculaire précédent et de l'intervalle d'échappement instantané que le dispositif aurait appliqué en l'absence de cette extrasystole auriculaire.

Par exemple, l'intervalle d'échappement auriculaire intermédiaire peut correspondre à la moyenne entre l'intervalle de temps séparant l'extrasystole auriculaire détectée du précédent événement auriculaire et l'intervalle moyen de la fréquence auriculaire.

De préférence, le dispositif comprend de plus des moyens pour diminuer l'intervalle d'échappement instantané (mode d'accélération programmé) et d'une façon avantageuse l'intervalle d'échappement instantané est diminué de façon itérative à chaque détection d'une séquence d'extrasystoles auriculaires jusqu'à une fréquence maximale prédéterminée ou indiquée par un capteur d'asservissement.

Si une phase d'accélération a été programmée sur l'apparition de l'extrasystole auriculaire, la fréquence de stimulation est une fonction du couplage de l'extrasystole et de l'intervalle d'échappement instantané accéléré calculé sur l'extrasystole.

Dans un mode de réalisation de l'invention, l'intervalle d'échappement instantané correspond à l'intervalle moyen moins un premier pourcentage si ledit intervalle moyen de la fréquence auriculaire est inférieur à un premier intervalle de temps et à l'intervalle moyen moins un deuxième pourcentage dans le cas contraire. De préférence, cet intervalle de temps est de 600 ms, le premier pourcentage est de 12 % et le deuxième pourcentage est de 6 %.

Selon un autre mode de réalisation de l'invention, des moyens de recyclage de l'intervalle d'échappement intermédiaire sont prévus pour recycler l'intervalle d'échappement intermédiaire si une nouvelle extrasystole auriculaire est détectée dans l'intervalle d'échappement intermédiaire. Le moyen de diminution de l'intervalle d'échappement instantané (IE) diminue ledit intervalle de façon itérative à chaque séquence de détection d'extrasystoles auriculaires (ESA) jusqu'à une fréquence maximale d'accélération (Fmacc) prédéterminée. Cette fréquence maximale d'accélération prédéterminée peut être programmée d'avance, calculée ou déterminée de toute façon appropriée.

De préférence cette fréquence maximale d'accélération correspond à la fréquence moyenne plus 30 cpm en dessous de 100 cpm ou à la fréquence moyenne plus 20 cpm au-dessus de ce seuil.

D'autres caractéristiques et avantages de l'invention apparaîtront à la lecture de la description détaillée ci-dessous en référence aux dessins annexés.
La figure 1 illustre le fonctionnement du dispositif selon l'invention en cas d'une extrasystole auriculaire isolée précoce sans accélération.
La figure 2 illustre le fonctionnement du dispositif selon l'invention en cas d'une extrasystole auriculaire isolée tardive sans accélération.
La figure 3 illustre le fonctionnement du dispositif selon l'invention en cas d'un doublet d'extrasystoles auriculaires précoces sans accélération.
La figure 4 illustre le fonctionnement du dispositif selon l'invention en cas d'un doublet d'extrasystoles auriculaires tardives sans accélération.
La figure 5 illustre le fonctionnement du dispositif selon l'invention en cas d'un triplet d'extrasystoles auriculaires précoces avec accélération.
La figure 6 illustre le fonctionnement du dispositif selon l'invention en cas d'un triplet d'extrasystoles auriculaires tardives avec accélération.

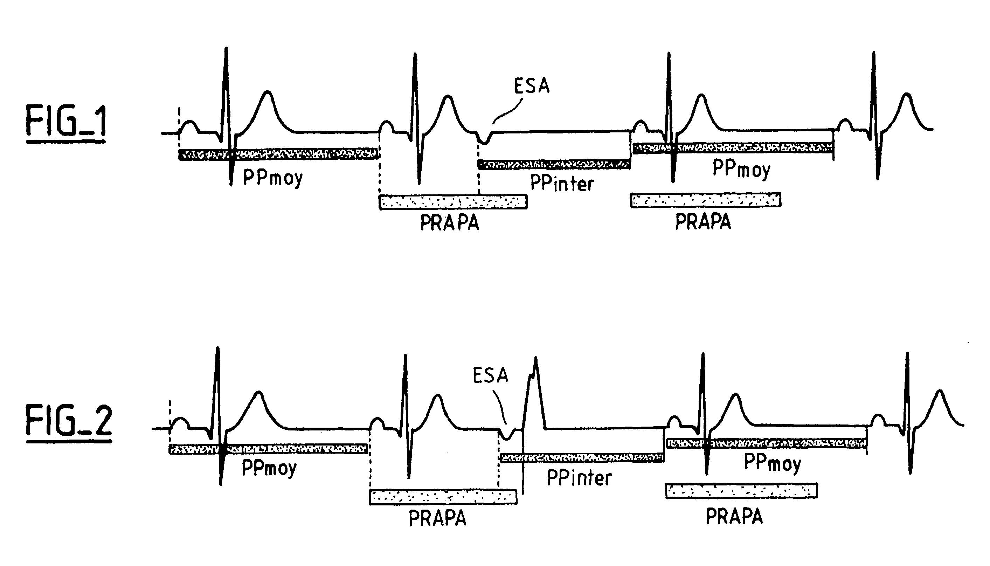

La figure 1 montre le principe de fonctionnement du dispositif selon l'invention dans le cas d'une extrasystole auriculaire isolée précoce.

Une onde ou un événement P (le recueil d'une activité spontanée ayant son origine dans l'oreillette) est définie comme une extrasystole auriculaire ESA si l'intervalle de temps séparant cette onde P du précédent événement auriculaire est inférieur à une fraction de l'intervalle moyen "PPmoy" de la fréquence auriculaire calculée, par exemple, sur huit cycles cardiaques antérieurs ne comprenant pas d'extrasystole. Dans les figures, PRAPA désigne la période de recherche de l'accélération post-auriculaire.

On définit qu'une extrasystole auriculaire est tardive si l'intervalle de temps séparant l'extrasystole auriculaire de l'onde P précédente est supérieur à 50 % de l'intervalle moyen PPmoy. Si cet intervalle est inférieur ou égal à ce taux, il s'agit d'une extrasystole auriculaire précoce.

Si l'extrasystole auriculaire est précoce (figure 1), on détermine un intervalle d'échappement intermédiaire "PPinter". Dans un premier mode de réalisation préféré, cet intervalle d'échappement intermédiaire PPinter correspond à la moyenne du couplage de l'extrasystole auriculaire et de l'intervalle d'échappement instantané IE calculé par le dispositif de façon en soi connue. L'intervalle d'échappement instantané IE est l'intervalle moyen PPmoy soit dans le cas de la figure 1 sans accélération programmée.

De préférence l'intervalle d'échappement instantané accéléré IEA vaut l'intervalle moyen PPmoy moins 12 % (de cette intervalle) si l'intervalle moyen PPmoy est inférieur à 600 ms et il vaut PPmoy moins 6 % dans le cas contraire.

Une variante consiste à pondérer de façon différente le couplage et PPmoy. De cette façon, la pause auriculaire qui aurait pu s'établir après l'apparition de l'extrasystole auriculaire est supprimée par la stimulation auriculaire qui a lieu à l'issue de l'intervalle d'échappement intermédiaire PPinter.

Dans le cas d'une extrasystole auriculaire tardive (figure 2), les moyens mis en oeuvre pour l'extrasystole auriculaire précoce restent appliqués. Mais de plus, on déclenche un délai auriculo-ventriculaire DAV, qui est le délai entre une onde P ou un événement A et une stimulation ventriculaire V suivante, si l'intervalle ventriculaire résultant de ce délai DAV est supérieur à une valeur programmable, par exemple de 400 ms. Dans le cas contraire, on ne déclenche pas de délai DAV.

Le fait de tenir compte du couplage de l'extrasystole auriculaire permet de réguler le rythme auriculaire de façon optimale. L'introduction d'un délai DAV en cas d'extrasystole auriculaire tardive stabilise de son côté le rythme ventriculaire et en résultat on obtient donc un lissage du rythme cardiaque et une meilleure adaptation physiologique.

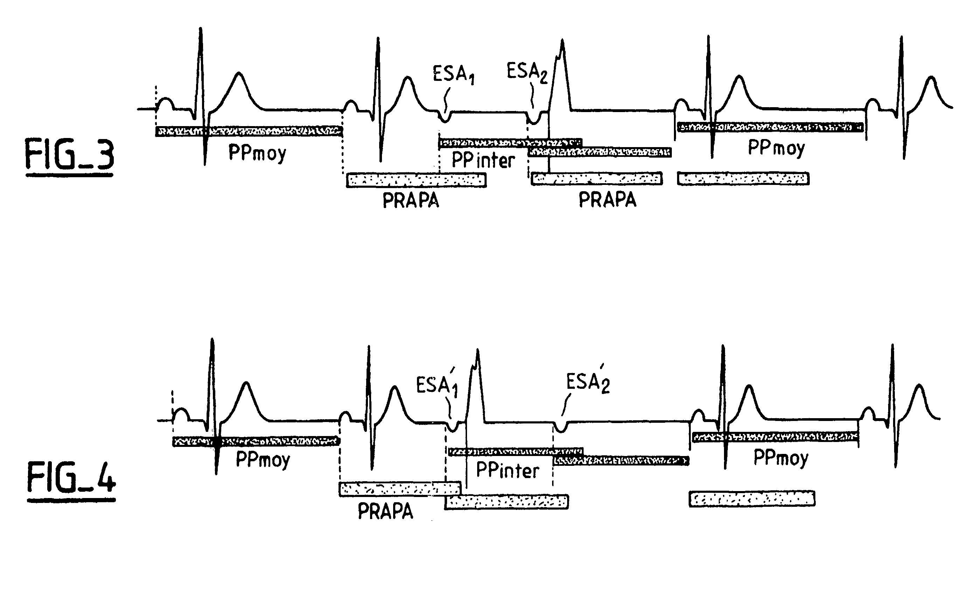

Dans le cas des doublets d'extrasystoles auriculaires (figures 3 et 4), la même distinction entre une extrasystole auriculaire précoce et tardive est faite.

La figure 3 montre le cas des doublets d'extrasystoles auriculaires précoces. Le calcul de l'intervalle d'échappement intermédiaire PPinter suit les mêmes lois que précédemment dans le cas d'une extrasystole auriculaire isolée. Sur une extrasystole auriculaire ESA₂ l'intervalle PPinter calculé avec le couplage de la première extrasystole auriculaire ESA₁ est recyclé sur la seconde extrasystole auriculaire ESA₂. Il n'est appliqué que sur la dernière extrasystole auriculaire de la salve, en l'occurrence la seconde extrasystole auriculaire ESA₂.

La figure 4 montre l'exemple des doublets d'extrasystoles tardives. Sur la première extrasystole auriculaire ESA'₁ de la salve qui est vue comme une extrasystole auriculaire isolée, le déclenchement du délai auriculo-ventriculaire DAV est géré comme dans le cas d'une extrasystole auriculaire isolée (voir ci-dessus). Pour l'extrasystole auriculaire suivante ESA'₂, l'intervalle d'échappement intermédiaire PPinter est recyclé. Il n'y a pas de nouveau déclenchement du délai DAV (fonctionnement Wenkebach en 2:1) et le dispositif stimule l'oreillette à l'issue du délai PPinter.

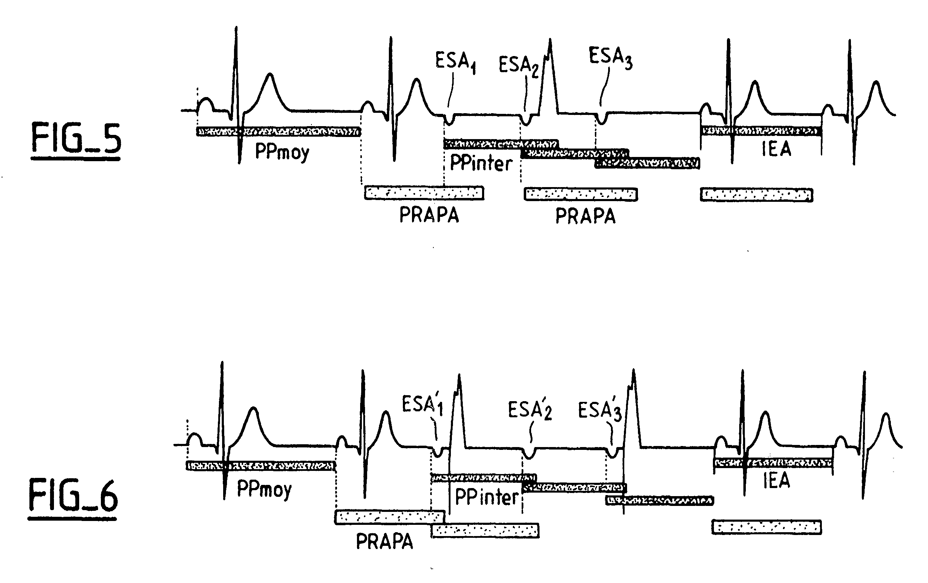

Le cas de triplets d'extrasystoles auriculaires constitue une généralisation de celui des doublets et est illustré sur les figures 5 et 6 avec programmation d'une phase d'accélération.

Les deux premières extrasystoles auriculaires ESA₁ et ESA₂ sont perçues par le dispositif comme un doublet et le délai PPinter est effectif sur la dernière extrasystole auriculaire ESA₃.

Dans un autre mode de réalisation préféré de l'invention et illustré pour le cas de triplets d'extrasystoles auriculaires sur les figures 5 et 6, sur chaque extrasystole auriculaire isolée (vraie extrasystole auriculaire isolée ou première extrasystole auriculaire d'une salve) et fréquente, l'intervalle d'échappement instantané IE, calculé par le dispositif, est diminué de 12 % si l'intervalle moyen PPmoy est inférieur à 600 ms, et de 6 % dans le cas contraire jusqu'à atteindre la fréquence maximale d'accélération Fmacc (l'intervalle d'échappement accéléré IEA). En l'absence d'extrasystole auriculaire fréquente, l'intervalle d'échappement se rallonge en suivant un lissage programmé tel que décrit dans la demande de brevet européenne EP-A-0 550 342. La fréquence maximale d'accélération Fmacc est évaluée en fonction du rythme courant. De préférence, elle vaut Fmoy (la fréquence correspondant à l'intervalle moyen PPmoy) plus 30 cpm au-dessous de 100 cpm et Fmoy plus 20 cpm au-dessus de ce seuil.

On se rapportera avantageusement au document EP-A-0 488 841 pour le calcul de l'intervalle d'échappement IE.

Toutefois, quand on a commencé à accélérer sur une extrasystole auriculaire on ne dispose plus de l'information sur le rythme cardiaque courant. La fréquence maximale d'accélération est alors contrôlée par l'information délivrée par un capteur prévu dans le dispositif selon l'invention. Tant que surviennent des extrasystoles auriculaires fréquentes on garde la fréquence maximale accélérée calculée à l'aide de la fréquence moyenne Fmoy avant le début de l'accélération sauf si le capteur indique une récupération ou sauf si la fréquence maximale d'accélération Fmacc_capt calculée en fonction de la fréquence obtenue par le capteur passe au-dessus de la fréquence maximale d'accélération Fmacc, la fréquence maximale d'accélération obtenue par le capteur étant évaluée de la même façon que la fréquence maximale d'accélération.

Dans ces deux derniers cas, on attribuera à la fréquence maximale d'accélération Fmacc la valeur de la fréquence maximale d'accélération Fmacc_capt obtenue par le capteur. Ainsi, on peut avoir une fréquence maximale Fmax qui correspond à l'état d'exercice physiologique du patient.

## Revendications

1. Dispositif médical implantable actif comprenant :
a) des moyens de détection des signaux d'activité cardiaque auriculaire,
b) des moyens de stimulation cardiaque dans les deux cavités cardiaques,
c) des moyens de détection de la survenue d'extrasystoles auriculaires (ESA),
d) des moyens pour déterminer et déclencher un intervalle d'échappement auriculaire intermédiaire (PPinter) appliqué lors de la détection d'une extrasystole auriculaire,
**caractérisé en ce que** l'intervalle d'échappement auriculaire intermédiaire (PPinter) est une fonction du couplage de l'extrasystole auriculaire (ESA) détectée par rapport à l'événement auriculaire précédent et de l'intervalle d'échappement instantané (IE) que le dispositif aurait appliqué en l'absence de cette extrasystole auriculaire (ESA).

2. Dispositif selon la revendication 1, **caractérisé en ce que** l'intervalle d'échappement auriculaire intermédiaire (PPinter) est la moyenne entre l'intervalle de temps séparant l'extrasystole auriculaire (ESA) détectée du précédent événement auriculaire et l'intervalle moyen (PPmoy) de la fréquence auriculaire.

3. Dispositif selon l'une des revendications précédentes, **caractérisé en ce que** des moyens de recyclage de l'intervalle d'échappement intermédiaire (PPinter) sont prévus pour recycler l'intervalle d'échappement intermédiaire (PPinter) si une nouvelle extrasystole auriculaire (ESA) est détectée dans l'intervalle d'échappement intermédiaire (PPinter).

4. Dispositif selon l'une des revendications précédentes, **caractérisé en ce qu'**il comprend de plus des moyens pour diminuer l'intervalle d'échappement instantané (IE) de façon itérative à chaque détection de séquence d'extrasystoles auriculaires (ESA) jusqu'à une fréquence maximale prédéterminée.

5. Dispositif selon la revendication 4, **caractérisé en ce que** le moyen de diminution de l'intervalle d'échappement instantané (IE) diminue ledit intervalle jusqu'à une fréquence maximale indiquée par un capteur d'asservissement.

6. Dispositif selon l'une des revendications 4 et 5, **caractérisé en ce que** l'intervalle d'échappement instantané (IE) correspond à l'intervalle moyen (PPmoy) moins un premier pourcentage, de préférence 12 %, si ledit intervalle moyen (PPmoy) de la fréquence auriculaire est inférieur à un intervalle de temps prédéterminé, de préférence 600 ms, et est l'intervalle moyen (PPmoy) moins un deuxième pourcentage, de préférence 6 %, dans le cas contraire.

7. Dispositif selon la revendication 5, **caractérisé en ce que** ladite fréquence maximale d'accélération (Fmacc) est la fréquence moyenne (Fmoy) plus 30 cpm en dessous d'une valeur typique de 100 cpm ou est la fréquence moyenne (Fmoy) plus 20 cpm au-dessus de cette valeur de 100 cpm.

## Patentansprüche

1. Aktive implantierbare medizinische Vorrichtung umfassend:
a) Mittel zur Detektion von Signalen der atrialen Herzaktivität,
b) Mittel zur Herzstimulation in den zwei Herzkammern,
c) Mittel zur Detektion des Auftretens von atrialen Extrasystolen (ESA),
d) Mittel zum Bestimmen und Auslösen eines atrialen Zwischenauslöseintervalls (PPinter), angewandt bei der Detektion einer atrialen Extrasystole,
**dadurch gekennzeichnet, dass** das atriale Zwischenauslöseintervall (PPinter) abhängig von der Kopplung der detektierten atrialen Extrasystole (ESA) im Verhältnis zu dem vorhergehenden atrialen Ereignis ist und dem augenblicklichen Auslöseintervall (IE), welches die Vorrichtung in Abwesenheit dieser atrialen Extrasystole (ESA) angewandt hätte.

2. Vorrichtung gemäß Anspruch 1, **dadurch gekennzeichnet, dass** das atriale Zwischenauslöseintervall (PPinter) das Mittel zwischen dem Zeitintervall ist, welches die detektierte atriale Extrasystole (ESA) von dem vorhergehenden atrialen Ereignis trennt, und dem mittleren Intervall (PPmoy) der atrialen Frequenz.

3. Vorrichtung gemäß einem der vorhergehenden Ansprüche, **dadurch gekennzeichnet, dass** die Mittel zum Wiederauslösen des Zwischenauslöseintervalls (PPinter) zum Wiederauslösen des Zwischenauslöseintervalls (PPinter) vorgesehen sind, wenn eine neue atriale Extrasystole (ESA) in dem Zwischenauslöseintervall (PPinter) detektiert wird.

4. Vorrichtung gemäß einem der vorhergehenden Ansprüche, **dadurch gekennzeichnet, dass** sie ferner Mittel zum Vermindern des augenblicklichen Auslöseintervalls (IE) umfasst, in iterativer Weise bei jedem Detektieren einer Sequenz von atrialen Extrasystolen (ESA) bis zu einer vorherbestimmten maximalen Frequenz.

5. Vorrichtung gemäß Anspruch 4, **dadurch gekennzeichnet, dass** das Mittel zur Verminderung des augenblicklichen Auslöseintervalls (IE) das Intervall vermindert, bis zu einer maximalen Frequenz, welche durch einen Regelungssensor angezeigt wird.

6. Vorrichtung gemäß einem der Ansprüche 4 und 5, **dadurch gekennzeichnet, dass** das unmittelbare Auslöseintervall (IE) dem mittleren Intervall (PPmoy) minus einem ersten Prozentsatz, vorzugsweise 12%, entspricht, wenn das mittlere Intervall (PPmoy) der atrialen Frequenz kleiner als ein vorherbestimmtes Zeitintervall ist, vorzugsweise 600 ms, und ist das mittlere Intervall (PPmoy) minus einem zweiten Prozentsatz, vorzugsweise 6%, in dem gegenteiligen Fall.

7. Vorrichtung gemäß Anspruch 5, **dadurch gekennzeichnet, dass** die maximale Beschleunigungsfrequenz (Fmacc) die mittlere Frequenz (Fmoy) plus 30 cpm unterhalb eines typischen Werts von 100 cpm ist oder die mittlere Frequenz (Fmoy) plus 20 cpm oberhalb dieses Werts von 100 cpm.

## Claims

1. Active implantable medical device, comprising:
a) means for detection of signals representative of atrial cardiac activity;
b) means for cardiac stimulation in the two cardiac cavities;
c) means for detection of the occurrence of atrial extrasystoles (ESA);
d) means for determining and releasing an intermediate atrial escape interval (PPinter) applied on detection of an atrial extrasystole;
**characterised in that** the intermediate atrial escape interval (PPinter) is a function of the coupling of the detected atrial extrasystole (ESA) as compared to the preceding atrial event and of the instantaneous escape interval (IE) that the device would have applied in the absence of said detected atrial extrasystole (ESA).

2. Device according to claim 1, **characterised in that** the intermediate atrial escape interval (PPinter) is the average value between the time interval separating the detected atrial extrasystole (ESA) from the preceding atrial event, and the average interval (PPmoy) of the atrial rate.

3. Device according to any of the preceding claims, **characterised in that** means for recycling the intermediate escape interval (PPinter) are provided for recycling the intermediate escape interval (PPinter) if another atrial extrasystole (ESA) is detected during the intermediate escape interval (PPinter).

4. Device according to any of the preceding claims, **characterised by** further comprising means for decreasing the instantaneous escape interval (IE) in an iterative manner on every detection of a sequence of atrial extrasystoles (ESA) up to a predetermined maximal rate.

5. Device according to claim 4, **characterised in that** the means for decreasing the instantaneous escape interval (IE) decreases said interval up to maximal rate set by an enslavement sensor.

6. Device according to any of claims 4 and 5, **characterised in that** the instantaneous escape interval (IE) corresponds to the average interval (PPmoy) of the atrial rate minus a first percentage, preferably 12 %, if said average interval (PPmoy) of the atrial rate is less than a predetermined time interval, preferably 600 ms, and is the average interval (PPmoy) minus a second percentage, preferably 6 %, in the opposite case.

7. Device according to claim 5, **characterised in that** said maximal acceleration rate (Fmacc) is the average rate (Fmoy) plus 30 bpm below a typical value of 100 bpm or is the average rate (Fmoy) plus 20 bpm below said value of 100 bpm.
